# FASCICULE DE BREVET EUROPEEN

(11) **EP 1 465 593 B1**
(45) Date de publication et mention de la délivrance du brevet: **19.08.2009**
(21) Numéro de dépôt: 02799831.9
(22) Date de dépôt: 19.12.2002
(51) Int. Cl.: A61K 9/00, A61K 9/16

(54) **FORME PHARMACEUTIQUE SOLIDE ORODISPERSIBLE**
IN DER MUNDHÖHLE DISPERGIERBARE FESTE PHARMAZEUTISCHE FORM
SOLID ORALLY-DISPERSIBLE PHARMACEUTICAL FORMULATION

(30) Priorité: 18.01.2002 FR 0200647
(43) Date de publication de la demande: 13.10.2004
(73) Titulaire: Roquette Frères, 62136 Lestrem (FR)
(72) Inventeur: SERPELLONI, Michel, F-62660 Beuvry-les-Béthune (FR)
(74) Mandataire: Boulinguiez, Didier
(86) Numéro de dépôt international: PCT/FR2002/004454
(87) Numéro de publication internationale: WO 2003/061623

(56) Documents cités:
- EP-A- 1 175 899
- CONTE, U.; ET AL: "On the direct compression of sulfamethoxydiazine (SMD) polymorphic forms" IL FARMACO EDIZIONE PRATICA, vol. 30, no. 4, avril 1975 (1975-04), pages 194-206, XP002212622 Pavia (IT)

## Description

L'invention a pour objet une forme pharmaceutique solide se désagrégeant rapidement dans la bouche.

Plus précisément, l'invention a pour objet une forme pharmaceutique solide orodispersible, comprenant au moins une substance active et un excipient particulier.

Même si la pharmacopée européenne 2001 ne recense pas encore la forme orodispersible parmi les nombreuses formes galéniques existantes, le terme « comprimé orodispersible » est aujourd'hui admis. Il répond à la définition suivante :
« Comprimé qui, placé dans la bouche, s'y disperse rapidement avant de l'avaler » (Pharmeuropa, vol.10,n°4, décembre 1998, p547).

La forme orodispersible a la particularité de ne nécessiter ni eau ni mastication au cours de son administration. Elle se désagrège en présence de salive, généralement en moins d'une minute.

Dans la conception de formes orodispersibles, deux formes sont réalisables :
- les formes qui s'effondrent : obtenues le plus souvent par lyophilisation, ces formes très poreuses contiennent des excipients solubles dont la dissolution provoque l'effondrement de la structure.
- les formes qui explosent : obtenues par compression, le mécanisme impliqué dans la désagrégation de ces formes dans la bouche est à imputer aux agents désintégrants qu'elles contiennent.

Dans la présente invention, on s'intéresse uniquement aux formes comprimées, et plus particulièrement aux formes obtenues par compression directe.

De nombreuses formes orales sont actuellement disponibles sur le marché mais elles ne correspondent pas toujours aux convenances des utilisateurs : les comprimés à avaler nécessitent l'absorption d'eau, les gommes et comprimés à mâcher impliquent une sollicitation des dents.

De nombreuses personnes ont des difficultés pour avaler les comprimés conventionnels souvent de taille non négligeable. Les problèmes liés à l'ingestion de médicaments (étouffement, suffocation par obstruction de la gorge) sont souvent à l'origine d'un mauvais respect des posologies, voire d'un arrêt du traitement. Ces problèmes concernent en particulier les enfants, les personnes âgées, les patients atteints de troubles de la déglutition ou de pathologies affectant la sécrétion salivaire.

Les formes orodispersibles tentent de pallier ces inconvénients par leur mode d'administration simple. Grâce à une désagrégation rapide, possible en présence de salive, le comprimé orodispersible est réduit, quelques dizaines de secondes après son administration, en agrégats de faible dimension, faciles à avaler. Dans le cas de la formulation d'une forme à libération d'actif immédiate, la dispersion orale permet une mise à disposition dans l'organisme plus rapide en comparaison avec les formes à avaler par augmentation de la surface d'échange avec les liquides physiologiques. Il en résulte une biodisponibilité de l'actif accrue.

La dispersibilité orale ne répond pas à un phénomène unique. Le mécanisme de la désagrégation aurait plusieurs origines, qui sont le gonflement de l'agent de désagrégation en présence de salive, le développement d'un réseau capillaire favorisé par la présence de pores au sein du comprimé, la tendance des particules à reprendre leur forme initiale, la chaleur dégagée par le mouillage des constituants qui augmente la pression de l'air, et la force de répulsion entre les particules au contact d'eau. Quelle que soit la théorie impliquée, la pénétration d'eau est la première phase de la désagrégation. Les constituants des comprimés orodispersibles doivent donc la favoriser ou au moins ne pas l'entraver. En termes de formulation et de fabrication, il s'agit donc de trouver un compromis entre caractéristiques physiques du comprimé et propriétés chimiques des excipients.

De nombreuses formes à dissolution rapide sont décrites dans l'art antérieur. U. Conte et al. (« On the direct compression of sulfamethoxydiazine (SMD) polymorphic forms » Il Farmatico Edizione Practica, vol. 30, no. 4, avril 1975, pages 194-203) décrit une forme solide pharmaceutique comprenant une substance active (sulfaméthoxydiazine) et des granules consistant en lactose et amidon. Le brevet US 5.464.632 décrit une technologie basée sur le pelliculage du principe actif, qui a pour but non seulement de masquer le goût des molécules actives mais aussi de créer un revêtement insoluble améliorant la vitesse de désagrégation du comprimé. En effet, la solubilisation des excipients en surface constitue un frein à la pénétration de l'eau dans le comprimé par augmentation de la viscosité du liquide entrant. La formule met en oeuvre un diluant (polyols), un agent désintégrant (polyvinylpyrrolidone réticulée) et les lubrifiants et adjuvants habituel.

Le document PCT/FR00/00495 décrit l'utilisation de lubrifiants hydrophobes dont l'action négative sur la pénétration de l'eau est compensée par l'utilisation d'un agent perméabilisant tel que la silice, afin d'augmenter l'affinité des comprimés pour l'eau. La formule comprend également un diluant et un agent désintégrant.

Le document WO 00/57857 décrit quant à lui l'utilisation d'un agent effervescent couplée avec un agent désintégrant de manière à améliorer la désagrégation en bouche. La formule comprend en outre un diluant non directement compressible.

Enfin, le document FR 98.09221 (FR 2 781 152) décrit une technologie basée sur la synergie entre un agent désintégrant et un polymère acrylique de type C, qui conduit à une vitesse de désagrégation grandement améliorée.

Toutes ces technologies ont en commun l'utilisation d'au moins un agent désintégrant encore appelé « super désintégrant ». Ce terme regroupe les composés dont le pouvoir désintégrant est élevé. Parmi les plus performants on connaît notamment le KOLLIDON^{®}CL (polyvinylpyrrolidone réticulée commercialisée par BASF), l'EXPLOTAB^{®} (fécule carboxyméthylée commercialisée par PENWEST), et l'AC DI SOL^{®} (carboxyméthylcellulose sodique réticulée commercialisée par FMC). Cet agent super désintégrant est indispensable dans la formulation de comprimés orodispersibles, et doit être utilisé conjointement avec un excipient de compression directe.

Le formulateur est alors contraint de préparer des mélanges physiques des différents excipients nécessaires à la formulation de comprimés orodispersibles. Or les mélanges physiques imposent des contraintes strictes sur leur réalisation et leur manipulation pour s'assurer d'une homogénéité du mélange et de l'absence de démélange, propriétés indispensables dans la pratique pour obtenir des comprimés de qualité constante, et ces mélanges ne permettent pas de préparer des comprimés de dureté variable en fonction de l'application visée. En effet, les résultats obtenus avec de tels mélanges donnent lieu à-des comprimés de dureté très élevée, tout à fait inadaptée à une désagrégation rapide dans la cavité buccale.

L'invention a donc pour but de remédier à cet inconvénient et de fournir une forme solide pharmaceutique orodispersible, à texture agréable, tout en mettant en oeuvre un excipient simple, d'origine naturelle, se désagrégeant rapidement dans la bouche, et présentant une neutralité gustative avantageuse.

La Demanderesse a eu le mérite de trouver que ce but pouvait être atteint dès lors qu'on met en oeuvre pour préparer ladite forme solide, un excipient particulier susceptible de remplir à la fois les fonctions de liant, désintégrant et diluant, tout en autorisant la préparation de formes solides sur une large gamme de duretés.

L'invention est définie par les revendications.

On entend par forme pharmaceutique solide au sens de la présente invention toute présentation sous forme de comprimés, obtenus par densification de poudre. Ces formes solides consistent essentiellement en matériaux inertes regroupés sous le terme d'excipients, et comprennent une ou plusieurs substances actives pharmaceutiques.

On entend par le terme « orodispersible » des formes solides qui se délitent dans la cavité buccale en moins de 3 minutes, et de préférence en moins d'une minute.

Lesdits granules compris dans les formes solides conformes à l'invention correspondent aux compositions décrites dans la demande de brevet EP 00/402159.8 dont la Demanderesse est titulaire. Ces granules sont caractérisés par une structure sphérique et une comprimabilité avantageuse et sont commercialisés sous l'appellation STARLAC^{®} par la Demanderesse et par la société MEGGLE GmbH.

Les propriétés désintégrantes desdits granules sont connues pour des comprimés placés dans des volumes de liquides importants, sous agitation. Il est particulièrement surprenant que de tels granules employés pour la fabrication de formes orodispersibles puissent donner des résultats particulièrement satisfaisants en terme de désagrégation en bouche, et ce pour deux raisons.

La première est basée sur le constat que les excipients les moins solubles dans l'eau sont les plus appropriés à la formulation de comprimés orodispersibles (la solubilisation, entraînant une augmentation de viscosité de l'eau, est un frein à sa pénétration dans les comprimés). Or lesdits granules comprennent une fraction importante de lactose très soluble dans l'eau. De plus, l'amidon compris dans lesdits granules n'est pas un agent « super désintégrant » tel qu'utilisé et décrit dans les formes orodispersibles de l'art antérieur.

La deuxième est basée sur le constat que les propriétés de désintégration d'un excipient (utilisé dans un comprimé) évaluées dans l'eau par les méthodes conventionnelles ne sont pas extrapolables au comportement du même comprimé in vivo, dans la salive. En effet, les vitesses de désintégration dans l'eau sont mesurées (selon la Pharmacopée Européenne) dans une quantité d'eau suffisamment importante pour ne pas atteindre la saturation en terme de solubilisation, alors que « in vivo », de par le faible volume de salive, les excipients sont à saturation. De plus, l'agitation à laquelle sont soumis les comprimés lors du test usuel ne reflète pas la désagrégation en bouche. La Demanderesse a ainsi constaté lors d'essais comparatifs que certains excipients connus comme bons désintégrants n'étaient pas adaptés à la préparation de formes orodispersibles. Inversement, certains excipients se désintégrant moyennement dans l'eau peuvent présenter des propriétés avantageuses in vivo.

La demanderesse a alors trouvé que lesdits granules conféraient de façon surprenante aux comprimés de très bonnes aptitudes à se désagréger en bouche, et ce pour une large gamme de duretés de comprimés, tout en conservant une friabilité faible ce qui est particulièrement remarquable. En effet, la plupart des formes orodispersibles de l'art antérieur qui se délitent rapidement dans la bouche sont très friables, ce qui se traduit par une désagrégation du comprimé dès qu'il est manipulé et ôté de son emballage.

La forme pharmaceutique orodispersible selon l'invention, c'est à dire se délitant en bouche en moins d'une minute, présente avantageusement une friabilité inférieure à 2%, et de préférence inférieure ou égale à 1%. Cette friabilité est mesurée selon la méthode de pharmacotechnie 2.9.7 de la pharmacopée européenne, 3^{ème} édition.

Il est particulièrement remarquable que les critères d'orodispersibilité et de friabilité faible précités soient respectés pour une large gamme de dureté de comprimés, c'est à dire pour des comprimés présentant une dureté mesurée à l'aide d'un duromètre de type ERWEKA TBH 30 GMD comprise entre 30 et 300 Newtons.

Les formes pharmaceutiques orodispersibles selon l'invention constituent donc pour les raisons qui viennent d'être exposées, un nouveau produit industriel.

Pour préparer ces formes pharmaceutiques selon l'invention, on s'y prend comme suit ou de manière équivalente.

On sélectionne tout d'abord la ou les substances actives souhaitées. Ces substances actives peuvent être choisies parmi un grand nombre d'actifs pharmaceutiques destinés à l'administration par voie orale, et en particulier dans le groupe constitué par les antalgiques, les antipyrétiques, les anti-diarrhéiques, les antispasmodiques, les anti-infectieux, les antibiotiques, les antiviraux, les antiparasitaires, les régulateurs de la motricité digestive, les régulateurs de la tension artérielle, de l'insuffisance cardiaque et coronarienne, les régulateurs du rythme cardiaque, les régulateurs du système nerveux central, les régulateurs des métabolismes lipidique, glucidique et protéique, du métabolisme osseux, les vasculoprotecteurs et veinotoniques, les agents régulateurs des systèmes hormonaux et immunitaires, les anti-inflammatoires stéroïdiens et non stéroïdiens, les antihistaminiques et antiallergiques, les antiasthmatiques, les antitussifs, les expectorants, les mucorégulateurs, les antiémétiques, les diurétiques, les laxatifs, les cytotoxiques et cytostatiques, les éléments vitaminiques et minéraux, les extraits végétaux.

Dans certains cas, on peut avantageusement masquer l'amertume ou le goût désagréable de la substance active, ou encore modifier son adsorption en procédant à un enrobage par pelliculage ou dragéification de ladite substance active. Le pelliculage peut être effectué avec divers agents de masquage de goût connus de l'homme de l'art, tels que notamment des polymères cellulosiques (éthylcellulose, hydroxyéthylcellulose, hydroxypropylméthylcellulose, hydroxypropyl cellulose, acétate de cellulose, acétophtalate de cellulose), les polyméthacrylates commercialisés sous l'appellation EUDRAGIT^{®}, des mélanges de polymères cellulosiques et d'autres polymères tels que par exemple la polyvinylpyrrolidone commercialisée sous l'appellation ROLLIDON^{®}, tous ces polymères ou leurs mélanges étant éventuellement associés à des plastifiants comme notamment le polyéthylène glycol.

Le pelliculage de la substance active est effectué selon un procédé connu tel que, par exemple, le procédé en lit d'air fluidisé, en turbine, la coacervation, la microencapsulation, l'extrusion-sphéronisation.

Bien que moins courante, on peut également utiliser la dragéification comme technique d'enrobage de la substance active. Celle-ci est conduite selon des procédés connus de l'homme de l'art, mettant en oeuvre divers sucres ou polyols éventuellement mélangés avec des polymères filmogènes.

La quantité de substance active présente dans la forme solide selon l'invention dépend de l'actif choisi. Généralement, celle-ci représente 0,2 à 95%, et préférentiellement 1 à 50% en poids de la forme solide.

On mélange cette substance active avec l'excipient consistant en granules d'amidon et de lactose.

De préférence, ces granules présentent un ratio lactose/amidon compris entre 90/10 et 25/75 et plus préférentiellement encore compris entre 85/15 et 50/50.

Ces granules peuvent être obtenus selon plusieurs variantes et en particulier par coséchage. De préférence, lesdits granules sont obtenus par coatomisation de lactose et d'amidon, selon un procédé décrit dans la demande de brevet précitée.

Les proportions desdits granules dans la forme solide selon l'invention varient en fonction du type de médicament que l'on souhaite préparer. En général, ces granules représentent de 20 à 99%, de préférence 40 à 98% en poids de ladite forme solide.

On peut également faire comporter au mélange ainsi préparé un ou plusieurs composés consistant en arômes, lubrifiants, colorants, édulcorants.

Selon une variante avantageuse de l'invention, on peut faire comporter au mélange jusqu'à 50% en poids d'un ou plusieurs édulcorants en poudre de manière à améliorer encore davantage les propriétés organoleptiques et mécaniques dudit mélange. Il va de soi que la quantité d'édulcorant est choisie de manière à ne pas entraver les propriétés d'orodispersibilité du comprimé.

Ces édulcorants peuvent consister en sucres tels que par exemple le dextrose, le maltose, le fructose, les maltodextrines, ou en polyols tels que le mannitol, le sorbitol, le maltitol, le xylitol, l'érythritol, le lactitol. De préférence, on utilisera des sucres ou polyols adaptés à la compression directe, et encore plus préférentiellement des polyols.

De très bons résultats ont été obtenus en faisant comporter audit mélange du mannitol pour compression directe, notamment du mannitol atomisé tel que le PEARLITOL^{®}200 SD commercialisé par la Demanderesse, et en particulier dans une proportion de 10 à 30%, de préférence 15 à 25% en poids.

On peut également faire comporter au mélange un couple effervescent, consistant par exemple en un acide organique et une base carbonatée de manière à préparer des comprimés orodispersibles présentant une légère effervescence. Ceci est particulièrement avantageux lorsqu'on cherche à optimiser l'aspect gustatif du comprimé. En effet, dès que celui-ci est placé en bouche, une légère effervescence se produit instantanément, comblant ainsi le temps de latence observé avant le commencement de la désagrégation du comprimé proprement dite. Le consommateur a donc une perception plus complète et plus agréable du délitement du comprimé en bouche.

A titre indicatif, on peut mettre en oeuvre environ 5% en poids de ce couple acide/base, consistant par exemple en acide citrique et bicarbonate de soude.

On procède ensuite à la mise en forme dudit mélange par densification de la poudre. En particulier, cette opération peut être réalisée par compression directe.

Les formes solides ainsi obtenues présentent une orodispersibilité tout à fait remarquable et ce, quelle que soit la dureté ou la densité de celles-ci. En effet, la mise en oeuvre des granules de lactose et amidon conformes à l'invention permet au formulateur de choisir parmi un panel très large de paramètres de fabrication de formes solides tout en étant assuré d'obtenir finalement une forme peu friable, se désagrégeant très rapidement dans la bouche, ce que ne permettaient pas les excipients de l'art antérieur destinés aux formes à libération rapide. De plus, les propriétés desdits granules permettent au formulateur de s'affranchir de l'ajout d'un super désintégrant dans la formule de comprimés, ce qui est très avantageux sur le plan technique et économique. L'utilisation selon l'invention de granules consistant en lactose et amidon coséchés dans la fabrication de formes orodispersibles se délitant en bouche en moins d'une minute est donc particulièrement innovante.

Les formes solides conformes à l'invention peuvent également trouver leur intérêt dans le domaine alimentaire et en particulier en confiserie. Il s'agira alors de remplacer l'actif pharmaceutique par toute autre substance alimentaire ou par une substance destinée par exemple à l'hygiène buccale.

L'invention sera mieux comprise à la lecture des exemples qui suivent et de la figure qui s'y rapporte, visant à illustrer de manière non limitative des modes de réalisation avantageux et à mettre en évidence la supériorité par rapport à l'art antérieur des formes solides conformes à l'invention.

### Exemple 1 : préparation de formes solides selon l'invention.

On prépare des comprimés selon l'invention, de dureté et de poids variables, dont on mesure le temps de délitement en bouche. Ce temps de délitement correspond au temps nécessaire pour que toute la suspension issue du délitement du comprimé placé en bouche soit avalée (le temps de déglutition fait partie intégrante du temps de délitement pour tenir compte des cas éventuels où une hygroscopicité trop élevée pourrait gêner la déglutition par manque de salive).

Le temps de délitement est mesuré par chronométrage : on déclenche le chronomètre dès que le comprimé est placé dans la bouche, on l'arrête lorsque toute la suspension est avalée.

La dureté des différents comprimés obtenus est mesurée à l'aide d'un duromètre de type ERWEKA TBH 30GMD.

Différentes substances actives sont utilisées : paracétamol, ibuprofène, vitamine C.

On utilise pour préparer les comprimés une presse de type FETTE EXACTA 21 équipée de poinçons plats.

### a)comprimés de paracétamol.

**TABLEAU 1**

| **constituants** | **Formule centésimale** | **Formule unitaire** |
|---|---|---|
| Paracétamol enrobé (RHODAPAP NCR) | 53,76% | 537,6mg |
| STARLAC^{®} | 44,44% | 444,4mg |
| Arôme fruits rouges | 1,00% | 10,0mg |
| aspartame | 0,30% | 3,0mg |
| Stéarate de magnésium | 0,5% | 5,0mg |
| TOTAL | 100 | 1000mg |

Caractéristiques des comprimés :
- diamètre = 16mm
- poids = 1g
- dureté Erweka = 45N
- temps de délitement dans la bouche < 30 secondes

### b)comprimés d'Ibuprofène.

**TABLEAU 2**

| **constituants** | **Formule centésimale** | **Formule unitaire** |
|---|---|---|
| Ibuprofène enrobé de matières grasses (70% de substance active en final) | 23,82% | 142,9mg |
| STARLAC^{®} | 73,18% | 439,1mg |
| Arôme fruits rouges | 2,00% | 12,0mg |
| aspartame | 0,5% | 3,0mg |
| Stéarate de magnésium | 0,5% | 3,0m |
| TOTAL | 100% | 600,0mg |

Caractéristiques des comprimés :
- diamètre = 13mm
- poids = 600mg
- dureté Erweka = 40N
- temps de délitement en bouche < 20 secondes

**TABLEAU 3**

| **constituants** | **Formule centésimale** | **Formule unitaire** |
|---|---|---|
| Ibuprofène enrobé de polysaccharide (90% de substance active en final) | 18,52% | 111,1mg |
| STARLAC^{®} | 78,48% | 470,9mg |
| Arôme fruits rouges | 2,00% | 12,mg |
| aspartame | 0,50% | 3,0m |
| Stéarate de magnésium | 0,5% | 3,0mg |
| TOTAL | 100% | 600,0mg |

Caractéristiques des comprimés :
- diamètre = 13mm
- poids = 600mg
- dureté Erweka = 30N
- temps de délitement en bouche < 20 secondes

### c) comprimés de Vitamine C.

**TABLEAU 4**

| **constituants** | **Formule centésimale** | **Formule unitaire** |
|---|---|---|
| Vitamine C | 25% | 100mg |
| STARLAC^{®} | 74.5% | 298mg |
| Stéarate de magnésium | 0,5% | 2,0mg |
| TOTAL | 100% | 400,0mg |

Caractéristiques des comprimés :
- diamètre = 10mm
- poids = 400mg
- dureté Erweka = 80N
- temps de délitement en bouche = 30 secondes

CONCLUSION : quelles que soient les caractéristiques des comprimés en termes de dureté, poids, diamètre, le temps de délitement en bouche est toujours faible, inférieur à 40 secondes et ce, même pour des duretés élevées. Ceci est particulièrement avantageux car des duretés importantes de comprimés autorisent leur manipulation sans risque de détérioration.

### Exemple 2 : préparation de formes solides selon l'invention et comparaison avec une composition de l'art antérieur.

Une formule selon l'invention (STARLAC^{®} + lubrifiant) est comparée à une formule selon l'art antérieur (mannitol pour compression directe + agent désintégrant + lubrifiant) pour préparer des formes orodispersibles.

La compression est effectuée sur presse FETTE EXACTA 21 équipée de poinçons plats de diamètre 16mm avec bords biseautés.

On mesure les différentes caractéristiques des comprimés obtenus :
- poids moyen
- épaisseur moyenne
- densité
- dureté Erweka
- friabilité selon la Pharmacopée Européenne
- temps de délitement en bouche
- force de compression nécessaire à l'obtention du comprimé

### Formule selon l'invention :

STARLAC^{®} : 99,4%
Stéarate de magnésium : 0,6%
Mélange : 5 minutes dans un mélangeur TURBULA

### Formule selon l'art antérieur :

Mannitol PARTECK^{®}M300 (MERCK) : 95,0%
KOLLIDON^{®}CL (BASF) : 3,0%
Stéarate de magnésium : 2,0%
Mélange en TURBULA 5 minutes sans lubrifiant, puis 5 minutes avec le lubrifiant.

Les caractéristiques des différents comprimés sont données dans les tableaux ci-dessous :

**TABLEAU 5**

| **comprimés selon l'invention** | **Essai 1** | **Essai 2** | **Essai 3** | **Essai 4** | **Essai 5** |
|---|---|---|---|---|---|
| Poids moyen (mg) | 929 | 1009 | 1069 | 1113 | 1135 |
| *Coefficient de variation (%)* | *0.16* | *0.07* | *0.08* | *0.10* | *0.08* |
| Epaisseur moyenne (mm) | 4.00 | 4.00 | 3.98 | 3.99 | 3.98 |
| *Coefficient de variation (%)* | *0.06* | *0.05* | *0 . 09* | *0.05* | *0.05* |
| Densité | 1.160 | 1.250 | 1.340 | 1.390 | 1.420 |
| Dureté Erweka | 32.1 | 64.1 | 145.2 | 209.7 | 300.5 |
| *Coefficient* de *variation* (%) | *17.83* | *5.65* | *6.13* | *4.52* | *2.20* |
| Friabilité (%) | 12.28 | 2.17 | 0.84 | 0.54 | 0.44 |
| Temps de délitement en bouche (s) | 14 | 18 | 23 | 26 | 31 |
| Force de compression (kN) | 13.4 | 23 | 33.9 | 43.3 | 54.7 |

**TABLEAU 6**

| **comprimés selon l'art antérieur** | **Essai 6** | **Essai 7** | **Essai 8** | **Essai 9** |
|---|---|---|---|---|
| Poids moyen (mg) | 946 | 991 | 1036 | 1074 |
| *Coefficient de variation (%)* | *0.21* | *0.39* | *0.20* | *0.23* |
| Epaisseur moyenne (mm) | 4.01 | 4.00 | 4.01 | 4.01 |
| *Coefficient de variation (%)* | 0.11 | 0.04 | *0.09* | 0.10 |
| Densité | 1.170 | 1.230 | 1.280 | 1.330 |
| Dureté Erweka | 61.1 | 121.1 | 188.6 | 289.9 |
| *Coefficient de variation (%)* | *6.67* | *18.88* | *13.29* | *5.33* |
| Friabilité (%) | 2.3 | 0.90 | 0.50 | 0.50 |
| Temps de délitement en bouche (s) | 42 | 78 | 174 | 340 |
| Force de compression (kN) | 20.0 | 27.0 | 38.4 | 51.4 |

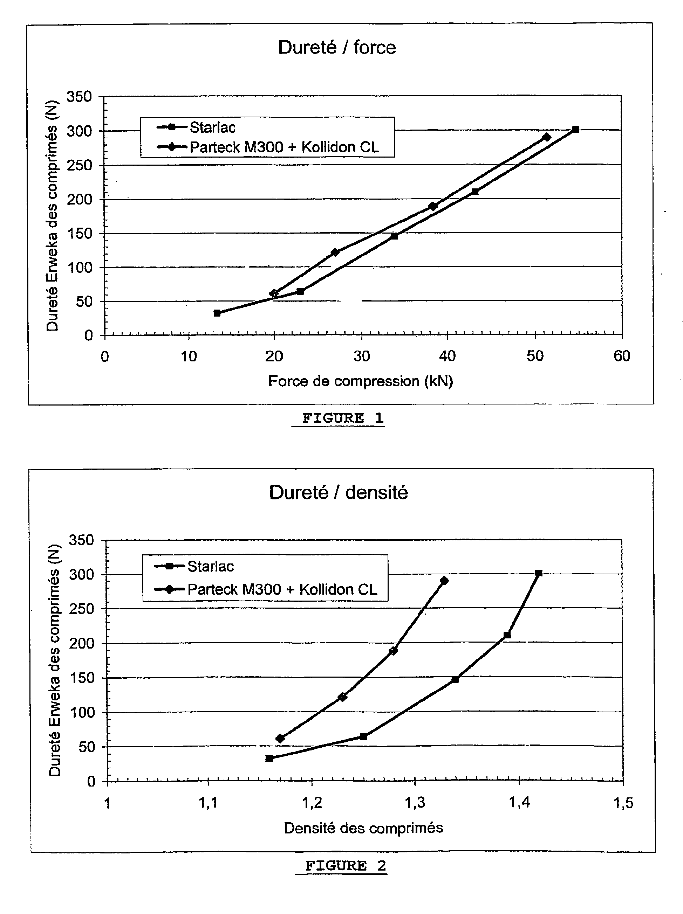

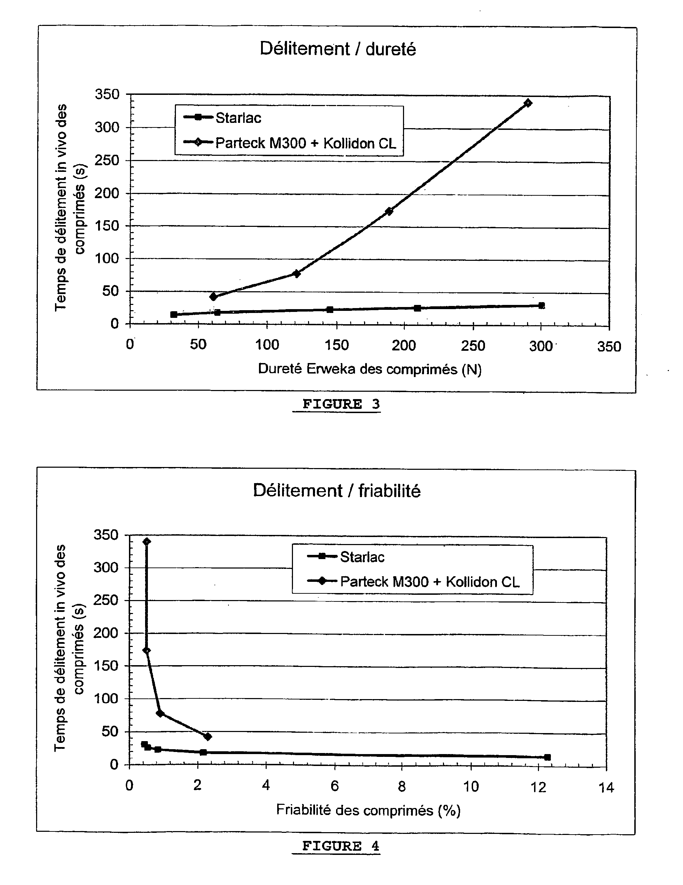

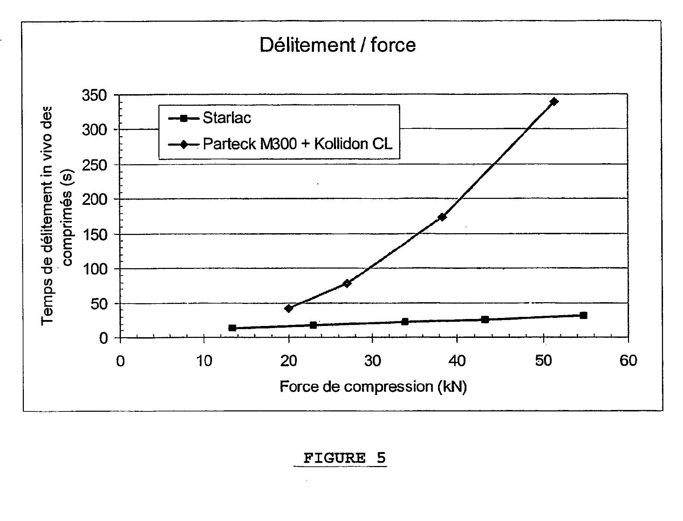

Il ressort de la comparaison des deux formulations que :
- la comprimabilité des deux formules est équivalente (ceci est illustré par la figure 1)
- quelles que soient les caractéristiques du comprimé fabriqué avec la composition selon l'invention (force de compression appliquée, dureté, densité, friabilité), le temps de délitement en bouche est toujours faible (inférieur à 40 secondes) et quasiment constant, ce qui est tout à fait remarquable. Ceci est illustré par les figures 2,3,4 et 5.
- Que la composition selon l'invention permet l'obtention d'un comprimé à délitement très rapide, ayant une dureté élevée et une friabilité faible (<1%). Ces caractéristiques autorisent la manipulation du comprimé dans des conditions standards, sans risque d'effritement, ce qui n'est pas le cas des comprimés de l'art antérieur qui ont une dureté faible et une friabilité élevée afin de conserver un délitement rapide en bouche.

### Exemple 3 : préparation de comprimés effervescents.

On prépare des comprimés orodispersibles conformes à l'invention, et présentant une légère effervescence, selon la formule suivante :

| | | |
|---|---|---|
| STARLAC^{®} | 580.8 mg | 72.6 % |
| PEARLITOL^{®}200 SD | 160.0 mg | 20.0% |
| Acide citrique anhydre | 16.0 mg | 2.0% |
| Bicarbonate de sodium | 20.0 mg | 2.5% |
| Colorant | 3.2 mg | 0.4% |
| Arôme orange | 8.0 mg | 1.0% |
| Aspartame | 4.0 mg | 0.5% |
| Acesulfame K | 4.0 mg | 0.5% |
| Stéarate de magnésium | 4.0 mg | 0.5% |

Le mélange est comprimé sur le même appareillage que précédemment, de manière à obtenir des comprimés de 5mm d'épaisseur et de diamètre 1,3cm.

Les comprimés se désagrègent totalement en bouche en 45 secondes, et présentent de plus une texture en bouche remarquable. L'ajout de mannitol pour compression directe n'entrave pas les propriétés d'orodispersibilité et améliore les qualités gustatives des comprimés.

## Revendications

1. Forme pharmaceutique solide orodispersible, **caractérisée en ce qu'**elle comprend :
- des granules consistant en lactose et amidon coséchés **caractérisés en ce que** le ratio lactose/amidon est compris entre 90/10 et 25/75, et de préférence entre 85/15 et 50/50, **en ce qu'**ils présentent une friabilité inférieure ou égale à 80% et de préférence à 60% selon un test A consistant à soumettre lesdits granules à une action mécanique dans un friabilimètre de marque ERWEKA TA équipé d'un tambour d'abrasion tournant à une vitesse de rotation uniforme de 25 tours/minute pendant 5 minutes dans lequel ont été introduites 5 billes d'acier identiques d'un diamètre de 17mm et d'un poids de 18,87 g et une quantité de 15 g desdits granules de granulométrie comprise entre 100 et 200 microns, la valeur de la friabilité correspondant au pourcentage de poudre non retenue sur un tamis d'une largeur de maille de 100 microns, et **en ce qu'**ils présentent une structure sphérique en microscopie électronique à balayage, susceptibles d'être obtenus par un procédé **caractérisé en ce qu'**il comprend une étape d'atomisation d'une suspension de lactose et d'amidon, - au moins une substance active.

2. Forme solide selon la revendication 1, **caractérisée en ce qu'**elle se délite dans la bouche en moins de 3 minutes et de préférence en moins d'une minute.

3. Forme solide selon l'une ou l'autre des revendications 1 et 2, **caractérisée en ce que** lesdits granules représentent 20 à 99%, de préférence 40 à 98% en poids de ladite forme solide.

4. Forme solide selon l'une quelconque des revendications 1 à 3, **caractérisée en ce que** lesdits granules présentent un ratio lactose/amidon de 85/15 à 50/50.

5. Forme solide selon l'une quelconque des revendications 1 à 4, **caractérisée en ce que** ladite substance active est choisie dans le groupe constitué par les antalgiques, les antipyrétiques, les anti- diarrhéiques, les antispasmodiques, les anti- infectieux, les antibiotiques, les antiviraux, les antiparasitaires, les régulateurs de la motricité digestive, les régulateurs de la tension artérielle, de l'insuffisance cardiaque et coronarienne, les régulateurs du rythme cardiaque, les régulateurs du système nerveux central, les régulateurs des métabolismes lipidique, glucidique et protéique, du métabolisme osseux, les vasculoprotecteurs et veinotoniques, les agents régulateurs des systèmes hormonaux et immunitaires, les anti-inflammatoiresstéroïdiens et non stéroïdiens, les antihistaminiques et antiallergiques, les antiasthmatiques, les antitussifs, les expectorants, les mucorégulateurs, les antiémétiques, les diurétiques, les laxatifs, les cytotoxiques et cytostatiques, les éléments vitaminiques et minéraux, les extraits végétaux.

6. Forme solide selon l'une quelconque des revendications 1 à 5, **caractérisée en ce que** ladite substance active représente 0,2 à 95%, et préférentiellement 1 à 50% en poids de la forme solide.

7. Forme solide selon l'une quelconque des revendications 1 à 6, **caractérisée en ce que** ladite substance active est enrobée par pelliculage ou dragéification.

8. Forme solide selon l'une quelconque des revendications 1 à 7, **caractérisée en ce qu'**elle présente une friabilité inférieure à 2%, de préférence inférieure ou égale à 1%, mesurée selon la méthode de pharmacotechnie 2.9.7 de la pharmacopée européenne, 3^{ème} édition.

9. Forme solide selon l'une quelconque des revendications 1 à 8, **caractérisée en ce qu'**elle comprend en outre un polyol pour compression directe.

10. Forme solide selon la revendication 9, **caractérisée en ce que** ledit polyol est le mannitol.

11. Utilisation de granules consistant en lactose et amidon tels que décrits à la revendication 1 dans la fabrication de formes pharmaceutiques orodispersibles se délitant en bouche en moins d'une minute.

## Claims

1. Orodispersible solid pharmaceutical form which comprises:
- granules consisting of lactose and starch which have been codried, wherein the lactose/starch ratio is comprised between 90/10 and 25/75, preferably between 85/15 and 50/50, wherein said granules have a brittleness of less than or equal to 80%, preferably 60%a according to a test A consisting of submitting said granules to a mechanical action in a ERWEKA TA friability tester, with an abrasion drum turning at a constant speed of 25 rpm during 5 minutes, in which have been introduced 5 identical steel balls, with a diameter of 17 mm and a weight of 18.87 g, and 15 g of said granules with a particle size of 100-200 micron, the value of the brittleness being the percentage of powder which has not been retained in a 100 micron sieve, and wherein said granules have a spherical structure under a scanning electron microscope, obtainable by a process **characterized by** the fact that it comprises a step of atomizing a lactose / starch suspension;
- at least one active substance.

2. The solid form of claim 1, which disintegrates in the mouth in less than 3 minutes and preferably in less than one minute.

3. The solid form of claim 1 or 2, wherein the said granules represent 20 to 99%, preferably 40 to 98% by weight of the said solid form.

4. The solid form of any of claims 1 to 3, wherein the said granules have a lactose/starch ratio of 85/15 to 50/50.

5. The solid form of any of claims 1 to 4, wherein the said active substance is selected from the group consisting of analgesics, antipyretics, antidiarrhoeals, antispasmodics, antiinfectious agents, antibiotics, antivirals, antiparasitics, digestive motility regulators, blood pressure regulators, cardiac and coronary insufficiency regulators, cardiac rhythm regulators, central nervous system regulators, lipid, carbohydrate and protein metabolism regulators, bone metabolism regulators, vasculoprotective and venotonic agents, hormone and immune system regulators, steroidal and nonsteroidal anti-inflammatory agents, antihistamines and antiallergics, antiasthmatics, antitusives, expectorants, mucoregulators, antiemetics, diuretics, laxatives, cytotoxics and cytostatics, vitamin and mineral elements, plant extracts.

6. The solid form of any of claims 1 to 5, wherein the said active substance represents 0.2 to 95%, and preferably 1 to 50% by weight of the solid form.

7. The solid form of any of claims 1 to 6, wherein the said active substance is coated by film-coating or hard-coating.

8. The solid form of any of claims 1 to 7, which has a brittleness of less than 2%, preferably of less than or equal to 1%, when measured according to the pharmaceutical technical method 2.9.7 of the European Pharmacopoeia, 3^{rd} edition.

9. The solid form of any of claims 1 to 8, which additionally comprises a polyol for direct compression.

10. The solid form of claim 9, wherein said polyol is mannitol.

11. Use of granules consisting of lactose and starch according to claim 1 for the manufacture of orodispersible pharmaceutical solid forms which disintegrate in the mouth in less than one minute.

## Patentansprüche

1. In der Mundhöhle dispergierbare feste pharmazeutische Form, **dadurch gekennzeichnet, dass** sie umfasst:
- Körnchen, die aus zusammen getrockneter Lactose und Stärke bestehen, **dadurch gekennzeichnet, dass** das Verhältnis Lactose/Stärke zwischen 90/10 und 25/75 ist und bevorzugt zwischen 85/15 und 50/50, dass sie eine Mürbigkeit von kleiner oder gleich 80% und bevorzugt von 60% aufweisen, gemäß einem Test A, der daraus besteht, dass die Körnchen einer mechanischen Beanspruchung in einem Friabilimeter der Marke ERWEKA TA, das mit einer Abriebtrommel ausgestattet ist, die sich mit einer einheitlichen Umdrehungsgeschwindigkeit von 25 Umdrehungen/Minute dreht, in das 5 identische Stahlkügelchen mit einem Durchmesser von 17 mm und einem Gewicht von 18,87 g und eine Menge von 15 g der Körnchen der Korngrößenbestimmung, die zwischen 100 und 200 Mikron aufweisen, eingefügt worden waren, für 5 Minuten ausgesetzt werden, wobei der Wert der Mürbigkeit dem Prozentsatz an Pulver entspricht, das nicht auf einem Sieb mit einer Maschenweite von 100 Mikron zurückgehalten wird, und dass sie im Rasterelektronenmikroskop eine kugelförmige Struktur aufweisen, erhältlich durch ein Verfahren, welches **dadurch gekennzeichnet ist, dass** es einen Sehritt der Zerstäubung einer Suspension aus Lactose und Stärke umfasst,
- mindestens einen Wirkstoff.

2. Feste Form nach Anspruch 1, **dadurch gekennzeichnet, dass** sie sich im Mund in weniger als 3 Minuten und bevorzugt in weniger als einer Minute auflöst.

3. Feste Form nach einem der Ansprüche 1 und 2, **dadurch gekennzeichnet, dass** die Körnchen 20 bis 99 Gewichts-%, bevorzugt 40 bis 98 Gewichts-% der festen Form darstellen.

4. Feste Form nach einem der Ansprüche 1 bis 3, **dadurch gekennzeichnet, dass** die Körnchen ein Verhältnis Lactose/Stärke von 85/15 bis 50/50 aufweisen.

5. Feste Form nach einem der Ansprüche 1 bis 4, **dadurch gekennzeichnet, dass** der Wirkstoff ausgewählt ist aus der Gruppe bestehend aus Antalgika, Antipyretika, Antidiarrhoika, Antispasmodika, Antiinfektiva, Antibiotika, Antivirenmitteln, Antiparasitika, Regulatoren der Verdauungstätigkeit, Regulatoren der Arterienspannung, der Herz- und Koronarinsuffienz, Regulatoren des Herzrythmus, Regulatoren des zentralen Nervensystems, Regulatoren der Lipid-, Kohlenhydrat- und Proteinmetabolismen, des Knochenmetabolismus, gefäßschützenden Mitteln und Venentonika, Regulationsmitteln von Hormon- und Immunsystemen, steroidalen und nicht-steroidalen antiinflammatorischen Mitteln, Antihistaminika und Antiallergika, Antiasthmatika, Antitussiva, Schleimlöser, Schleimregulatoren, Antiemetika, Diuretika, Laxantien, zytotoxischen und zytostatischen Mitteln, Vitaminen und Mineralstoffen, Pflanzenextrakten.

6. Feste Form nach einem der Ansprüche 1 bis 5, **dadurch gekennzeichnet, dass** der Wirkstoff 0,2 bis 95 Gewichts-% und bevorzugt 1 bis 50 Gewichts-% der festen Form darstellt.

7. Feste Form nach einem der Ansprüche 1 bis 6, **dadurch gekennzeichnet, dass** der Wirkstoff durch eine Beschichtung oder Dragierung umhüllt ist.

8. Feste Form nach einem der Ansprüche 1 bis 7, **dadurch gekennzeichnet, dass** sie eine Mürbigkeit von kleiner als 2%, bevorzugt kleiner oder gleich 1 % aufweist, gemessen gemäß dem pharmakotechnischen Verfahren 2.9.7 aus dem Europäischen Arzneibuch, 3. Auflage,

9. Feste Form nach einem der Ansprüche 1 bis 8, **dadurch gekennzeichnet, dass** es weiterhin ein Polyol für die direkte Verdichtung enthält.

10. Feste Form nach Anspruch 9, **dadurch gekennzeichnet, dass** das Polyol Mannitol ist.

11. Verwendung von Körnchen, die aus Lactose und Stärke bestehen, wie in Anspruch 1 beschrieben, zur Herstellung von in der Mundhöhle dispergierbaren pharmazeutischen Formen, die sich im Mund in weniger als einer Minute auflösen.
